# EUROPÄISCHE PATENTANMELDUNG

(11) **EP 0 554 219 A1**
(43) Veröffentlichungstag der Anmeldung: **04.08.1993**
(21) Anmeldenummer: 93810036.9
(22) Anmeldetag: 20.01.1993
(51) Int. Cl.: C08K 5/45, C08K 5/48, C07D 339/06, C07D 345/00, H01B 1/12, C08J 5/18, H01J 1/05

(54) **Kunststoffzusammensetzung mit Charge-Transfer Komplexen, deren Herstellung und deren Verwendung**

(30) Priorität: 29.01.1992 CH 242/92
(71) Anmelder: CIBA-GEIGY AG, CH-4002 Basel (CH)
(72) Erfinder: Chetcuti, Peter, Dr., CH-4052 Basel (CH)

(57) **Zusammenfassung**

Zusammensetzung, enthaltend a) ein duroplastisches, thermoplastisches oder strukturell vernetztes Polymer und b) einen Charge-Transfer Komplex der Formel I in Form eines Netzwerks aus Kristallnadeln in der Polymermatrix,

[A]^{⊖·}B^{⊕} (I),

worin
A eine Verbindung der Formel II oder eine Mischung von Verbindungen der Formel II ist, 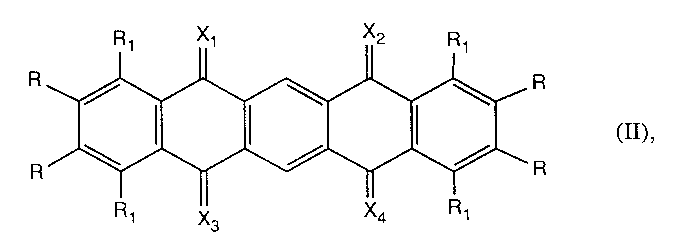 worin die R gleich sind und für H oder C₁-C₄-Alkyl stehen, oder die benachbarten R zusammen -(CH₂)₃- oder -(CH₂)₄- darstellen; R₁ H oder C₁-C₄-Alkyl bedeutet; X₁ =N-CN darstellt und X₂, X₃ und X₄ unabhängig voneinander =O oder =N-CN bedeuten, und
B eine Verbindung der Formel III oder IIIa bedeutet,

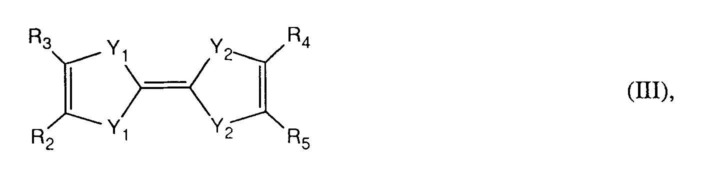

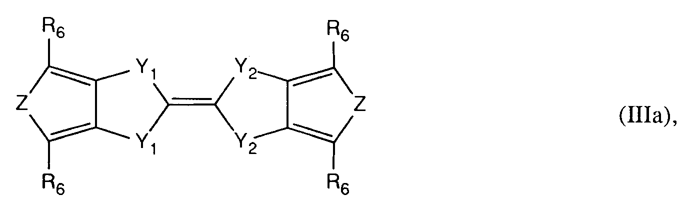

worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder NR₇ bedeutet und R₇ H, C₁-C₆-Alkyl, Phenyl oder Benzyl ist, und R₆ für H, C₁-C₄-Alkyl, Phenyl oder Benzyl steht. Diese Zusammensetzungen sind elektrische Leiter.

## Beschreibung

Die Erfindung betrifft Zusammensetzungen aus einem Kunststoff und einem Charge-Transfer Komplex (nachfolgend als CT-Komplex abgekürzt) aus Pentacencyanoiminderivaten als Elektronenakzeptoren und chalkogenierten Fulvalenen als Elektronendonatoren; ein Verfahren zu deren Herstellung; und die Verwendung der Zusammensetzungen als elektrische Leiter, zum Beispiel zur Herstellung von elektrisch leitenden Filmen, Folien oder Beschichtungen. Bei diesen CT-Komplexen handelt es sich um Radikalkationensalze.

In Synthetic Metals, 41-43, Seiten 2365 bis 2375 (1991) werden pulverförmige CT-Komplexe aus Tetracyanoiminpentacen und Tetrathiofulvalen als Elektronendonator beschrieben, die eine elektrische Leitfähigkeit von etwa 6 S/cm aufweisen. Pulverförmige Materialien können die elektrischen Leitfähigkeiten von Kunststoffen jedoch nur ungenügend verbessern, da die Teilchen nach der Einarbeitung vom Kunststoff umhüllt und damit isoliert werden.

Im US Patent 5,009,812 werden antistatisch ausgerüstete und elektrisch leitfähige Polymere beschrieben, die zum Beispiel CT-Komplexe aus Tetrathio-, Tetraseleno- oder Tetratellurotetracenen als Elektronendonatoren und Halogenen oder Sauerstoff als Elektronenakzeptoren enthalten. Die CT-Komplexe bilden in diesen Materialien Nadelnetzwerke in der Polymermatrix. Bei der Herstellung dieser elektrisch leitenden Polymeren müssen auf metallische Maschinenteile korrosiv wirkende Reaganzien verwendet werden, was besondere Schutzmassnahmen für die Maschinen erforderlich macht. Auf Grund der Schwerlöslichkeit chalkogenierter Tetracene müssen zudem bei der Herstellung relativ hohe Temperaturen eingestellt werden, was als unwirtschaftlich empfunden wird und wegen einer zu hohen Flüchtigkeit verwendeter Reaganzien auch arbeitshygenische Massnahmen erfordert. Auch die Verwendung von Tetraseleno- und Tetratellurotetracenen wird aus toxikologischen Gründen als bedenklich angesehen.

Es wurde nun gefunden, dass Pentacencyanoimine und bestimmte Fulvalenderivate überraschend CT-Komplexe bilden, die unerwartet selbst in Gegenwart von Bindemitteln nadelförmig kristallisieren, eine hohe elektrische Leitfähigkeit aufweisen und praktisch keine korrosive Wirkung auf metallische Teile von Verarbeitungsmaschinen zeigen. Die Ausgangsverbindungen sind auch in weniger polaren organischen Lösungsmitteln löslich, so dass zur Herstellung der CT-Komplexe keine sehr hohen Temperaturen angewendet werden müssen. Die CT-Komplexe weisen eine hohe Stabilität gegenüber Feuchtigkeit und Wärme auf.

Ein Gegenstand der Erfindung ist eine Zusammensetzung, enthaltend a) ein duroplastisches, thermoplastisches oder strukturell vernetztes Polymer und b) einen Charge-Transfer Komplex der Formel I in Form eines Netzwerks aus Kristallnadeln in der Polymermatrix,

[A]^{⊖·}B^{⊕} (I),

worin
A eine Verbindung der Formel II oder eine Mischung von Verbindungen der Formel II ist, 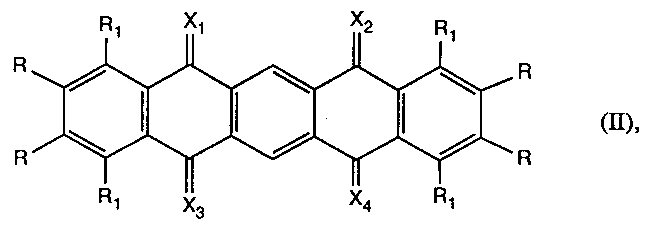
worin die R gleich sind und für H oder C₁-C₄-Alkyl stehen, oder die benachbarten R zusammen -(CH₂)₃- oder -(CH₂)₄- darstellen; R₁ H oder C₁-C₄-Alkyl bedeutet; X₁ =N-CN darstellt und X₂, X₃ und X₄ unabhängig voneinander =O oder =N-CN bedeuten, und
B eine Verbindung der Formel III oder IIIa bedeutet, 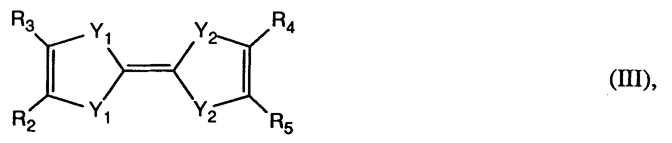 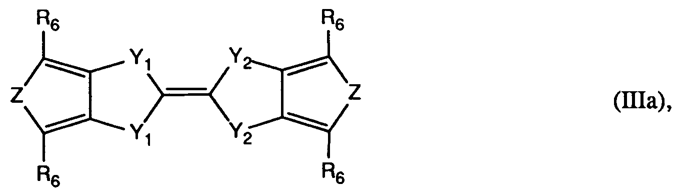
worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder NR₇ bedeutet und R₇ H, C₁-C₆-Alkyl, Phenyl oder Benzyl ist, und R₆ für H, C₁-C₄-Alkyl, Phenyl oder Benzyl steht.

Bei R und R₁ in der Bedeutung von Alkyl kann es sich um Methyl, Ethyl, n- oder i-Propyl oder n-, i- oder t-Butyl handeln. Bevorzugte Alkylreste sind Methyl und Ethyl. In einer bevorzugten Ausführungsform stellen die R C₁-C₄-Alkyl und die R₁ H dar, oder stellen die R₁ C₁-C₄-Alkyl und die R H dar. Bevorzugt stellen R und R₁ H, Methyl oder Ethyl dar. In einer besonders bevorzugten Ausführungsform bedeuten R und R₁ H.

In einer anderen bevorzugten Ausführungsform bedeuten X₁ und X₄ =N-CN und X₂ und X₃ =O oder =N-CN, oder X₂ und X₃ =N-CN und X₁ und X₄ =O oder =N-CN. Besonders bevorzugt stellen X₁, X₂, X₃ und X₄ =N-CN dar.

Die Verbindung der Formel II ist bevorzugt 5,7,12,14-Tetracyanoiminpentacen, das in reiner Form vorliegt, oder bis zu 10 Gew-%, bezogen auf die Gesamtmischung, Verbindungen der Formel II enthält, in denen ein oder zwei Cyanoimingruppen durch Sauerstoff ersetzt sind.

Die Verbindungen der Formeln III und IIIa weisen bevorzugt ein Reduktionspotential von kleiner oder gleich 0,44 V auf, bezogen auf die Standardkalomelelektrode.

In den Verbindungen der Formel III stellen R₂ und R₃ sowie R₄ und R₅ bevorzugt gleiche Reste dar und besonders bevorzugt handelt es sich bei R₂ bis R₅ um gleiche Reste.

In einer bevorzugten Ausführungsform handelt es sich bei Y₁ und Y₂ in den Verbindungen der Formel III und IIIa entweder um -S- oder um -Se-, und besonders bevorzugt um -S-.

Bei R₂ bis R₅ in der Bedeutung von Alkyl-(Z₁)ₙ- handelt es sich bevorzugt um C₁-C₁₂-Alkyl-(Z₁)n-, besonders bevorzugt um C₁-C₈-Alkyl-(Z₁)-ₙ und insbesondere bevorzugt um C₁-C₄-Alkyl-(Z₁)-ₙ. Bevorzugt ist das Alkyl linear. Beispiele für Alkyl sind Methyl, Ethyl, n- und i-Propyl, n-, i- und t-Butyl, Pentyl, Hexyl, Heptyl, Octyl, Nonyl, Decyl, Undecyl, Dodecyl, Tetradecyl, Hexadecyl und Octadecyl. Besonders bevorzugt sind Methyl, Ethyl, n- und i-Propyl, n-, i- und t-Butyl.

In einer bevorzgten Ausgestaltung handelt es sich bei dem Rest Alkyl-(Z₁)ₙ- um Methyl, Ethyl, n- und i-Propyl, n-, i- und t-Butyl, Methylthio, Methylseleno, Ethylthio und Ethylseleno.

Bei den Substituenten C₁-C₄-Alkyl, C₁-C₄-Alkoxy und C₁-C₄-Alkylthio kann es sich zum Beispiel um Methyl, Ethyl, n- und i-Propyl, n-, i- und t-Butyl sowie um entsprechende Alkoxy und Alkylthioreste handeln. Bevorzugte Substituenten sind Methyl, Ethyl, n- und i-Propyl, n- und i-Butyl, Methoxy, Ethoxy, Methylthio und Ethylthio.

Einige Beispiele für die Reste Phenyl-(Z₁)ₙ- oder Benzyl-(Z₁)ₙ- sind Phenyl, Benzyl, Phenylthio, Phenylseleno, Benzylthio, Benzylseleno, Methylphenyl, Methylbenzyl, Ethylphenyl, n- oder i-Propylphenyl, n-, i- oder t-Butylphenyl, Dimethylphenyl, Dimethylbenzyl, Methoxyphenyl, Methylthiophenyl, Methylthiobenzyl, Methylphenylthio und Methylphenylseleno.

In Formel IIIa stellt stellt R₆ bevorzugt H oder C₁-C₄-Alkyl dar, und besonders bevorzugt ist R₆ H, Methyl oder Ethyl.

Z in Formel llIa steht bevorzugt -S- oder -NR₇- und besonders bevorzugt für -NR₇-. R₇ bedeutet bevorzugt H oder C₁-C₄-Alkyl, und besonders bevorzugt ist R₇ H, Methyl oder Ethyl.

Z₁ bedeutet bevorzugt -S- und Z₂ stellt bevorzugt -O- oder -S-.

Eine bevorzugte Untergruppe der Verbindungen der Formel I sind solche, worin in Formel II R H, Methyl oder Ethyl und besonders bevorzugt H bedeutet, R₁ H oder Methyl und bevorzugt H darstellt und X₁ bis X₄ für =N-CN stehen, und in den Formeln III und IIIa R₂ und R₃ sowie R₄ und R₅ oder R₂ bis R₅ gleich sind und H, lineares oder verzweigtes C₁-C₈-Alkyl-(Z₁)ₙ-, unsubstituiertes oder mit C₁-C₄-Alkyl substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄Alkyl ist, und R₆ für H oder C₁-C₄-Alkyl steht.

Eine besonders bevorzugte Untergruppe der Verbindungen der Formel I sind solche, worin in Formel II R und R₁ H bedeuten, X₁ bis X₄ für =N-CN stehen, und in den Formeln III und IIIa R₂ bis R₅ gleich sind und H, oder lineares oder verzweigtes C₁-C₄-Alkyl-(Z₁)ₙ- darstellen, oder R₂ und R₃ sowie R₄ und R₅ je zusammen Trimethylen, Tetramethylen, -Z₂-(CH₂)-Z₂-, -Z₂-(CH₂)₂-Z₂-, -Z₁-CH=CH-Z₁- oder -CH=CH-CH=CH- bedeuten, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄-Alkyl ist, und R₆ für H oder C₁-C₄-Alkyl steht.

In einer besonders bevorzugten Ausführungsform enthält die erfindungsgemässe Zusammensetzung solche Verbindungen der Formel I, worin A 5,7,12,14-Tetracyanoiminpentacen ist und B eine Verbindung der Formel IIIb darstellt, 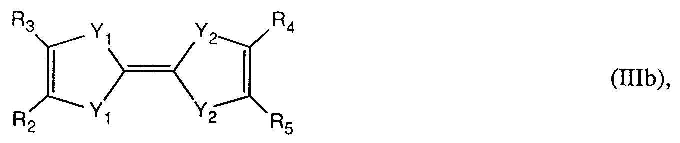
worin Y₁ und Y₂ je für -S- stehen, und R₂, R₃, R₄ und R₅ unabhängig voneinander für H, C₁-C₄-Alkyl oder C₁-C₄-Alkylthio oder R₂ und R₃ sowie R₄ und R₅ zusammen für -S-CH₂CH₂-S- stehen. Besonders bevorzugt sind in Formel IIIb R₂ und R₃ sowie R₄ und R₅ gleiche Reste, und ganz besonders bevorzugt sind R₂ bis R₅ gleiche Reste.

Einige Beispiele für CT-Komplexe der Formel I sind solche, worin A in Formel I 5,7,12,14-Tetracyanoiminpentacen ist, und B Tetrathiofulvalen, Tetramethyltetrathiofulvalen, Tetraethyltetrathiofulvalen, Dimethyl-diethyl-tetrathiofulvalen, Tetra-n-propyl-tetrathiofulvalen, Tetra-n-butyl-tetrathiofulvalen, Tetra(methylthio)tetrathiofulvalen, Tetra(ethylthio)tetrathiofulvalen, Tetra(n-propylthio)tetrathiofulvalen, Tetra(n-butylthio)tetrathiofulvalen, Dimethyl-dimethylthiotetrathiofulvalen, Diethyl-dimethylthiotetrathiofulvalen, Diethylthio-dimethylthiotetrathiofulvalen und Tetraselenofulvalen bedeutet.

Ein anderer Gegenstand der Erfindung ist ein Verfahren zur Herstellung von CT-Komplexen der Formel I, das dadurch gekennzeichnet ist, dass man äquimolare Mengen eines Fulvalenderivates B und eines Pentacencyanoimins der Formel II in einem inerten organischen Lösungsmittel umsetzt. Äquimolare Mengen bedeutet, dass man zur Bildung der 1:1-Komplexe etwa 1 Äquivalent des Fulvalenderivats der Formeln III oder IIIa mit etwa 1 Äquivalent des Pentacencyanoimins der Formel II umsetzt.

Die Fulvalenderivate sind bekannt, teilweise käuflich oder sie können nach allgemein bekannten Verfahren hergestellt werden. Die Herstellung von 5,7,12,14-Tetracyanoiminpentacen ist von L. L. Miller in Synthetic Metals, 41-43, Seiten 2365-2375 (1991) beschrieben. Die als Ausgangsverbindungen verwendeten unsubstituierten oder substituierten 5,7,12,14-Pentacentetrone sind nach einem von W. H. Mills et al. in J. Chem. Soc. 101, Seite 2194 (1912) beschriebenen Verfahren erhältlich. Die 5,7,12,14-Tetracyanoiminpentacene können nach üblichen Methoden gereinigt werden, zum Beispiel durch Umkristallisation oder chromatographische Verfahren. Wenn keine besonderen Schutzmassnahmen ergriffen werden, wie zum Beispiel wasserfreie Bedingungen, können Cyanoimingruppen durch Sauerstoff ersetzt werden, was aber die Bildung der gewünschten CT-Komplexe nicht negativ beeinflusst.

Das erfindungsgemässe Verfahren wird vorteilhaft bei erhöhten Temperaturen durchgeführt, zum Beispiel bei 30 bis 200°C, bevorzugt bei 50 bis 100°C.

Geeignete Lösungsmittel sind zum Beispiel unpolare, polare und aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol), Nitrile (Acetonitril, Propionitril) und aliphatische oder cycloaliphatische Kohlenwasserstoffe (Petrolether, Pentan, Hexan, Cyclohexan und Methylcyclohexan). Geeignet sind auch aromatisch-aliphatische Ether wie zum Beispiel Methyl- oder Ethylphenylether, und Ketone wie zum Beispiel Aceton, Methylethylketon, Methylpropylketon, Dipropylketon, Dibutylketon und Methylisobutylketon.

Die nach dem erfindungsgemässen Verfahren erhältlichen CT-Komplexe fallen in hohen Reinheiten an und brauchen nach der Filtration nur noch mit Lösungsmitteln gewaschen werden. Es handelt sich in der Regel um dunkelfaarbige bis schwarze Kristalle mit nadelförmiger Kristallgestalt, die elektrische Leitfähigkeiten von mehr als 0,1 S/cm aufweisen. Sie eignen sich hervorragend als elektrische Leiter. So können mit der Einarbeitung dieser CT-Komplexe in Kunststoffe je nach Art des CT-Komplexes und der verwendeten Menge elektrisch leitende oder antistatisch ausgerüstete Kunststoffe erhalten werden, wobei der CT-Komplex als Netzwerk der Kristallnadeln in der Kunststoffmatrix vorliegt. Je nach der Konzentration des CT-Komplexes in der Kunststoffmatrix können sehr feinmaschige Nadelnetzwerke erhalten werden.

Ein weiterer Gegenstand der Erfindung sind CT-Komplexe der Formel Ia

[A]^{⊖·}B^{⊕} (Ia),

worin
A eine Verbindung der Formel II oder eine Mischung von Verbindungen der Formel II ist, 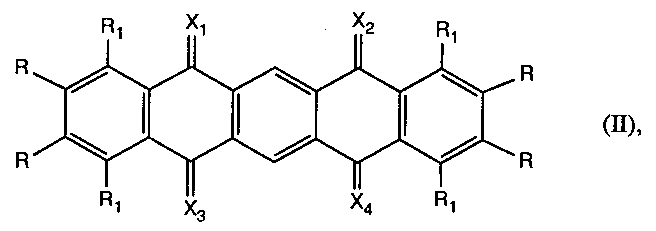
worin die R gleich sind und für H oder C₁-C₄-Alkyl stehen, oder die benachbarten R zusammen -(CH₂)₃- oder -(CH₂)₄- darstellen; R₁ H oder C₁-C₄-Alkyl bedeutet; X₁ =N-CN darstellt und X₂, X₃ und X₄ unabhängig voneinander =O oder =N-CN bedeuten, und B eine Verbindung der Formel III oder IIIa bedeutet, 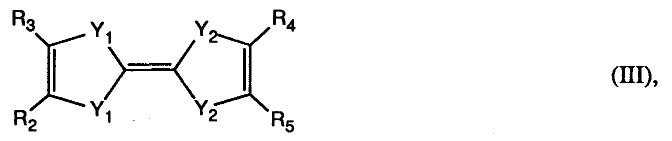 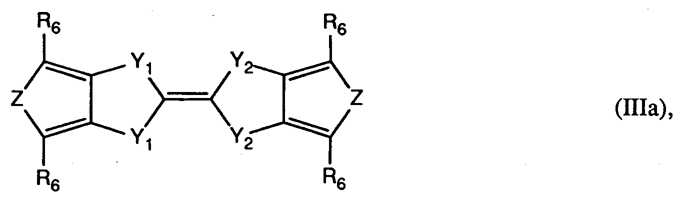
worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder NR₇ bedeutet und R₇ H, C₁-C₆-Alkyl, Phenyl oder Benzyl ist, und R₆ für H, C₁-C₄-Alkyl, Phenyl oder Benzyl steht, mit Ausnahme von [(5,7,12,14-Tetracyanoimintetracen)(Tetrathiofulvalen)].

Ein weiterer Gegenstand der Erfindung sind die CT-Komplexe der Formel I mit Ausnahme von [(5,7,12,14-Tetracyanoimintetracen)(Tetrathiofulvalen)] in Form von nadelförmigen Kristallen.

Für die erfindungsgemässen CT-Komplexe der Formel I gelten die gleichen Bevorzugungen und beispielhaften Ausgestaltungen, wie sie zuvor für die CT-Komplexe in den erfindungsgemässen Zusammensetzungen erwähnt werden.

In den erfindungsgemässen Zusammensetzungen können die CT-Komplexe in einer Menge von 0,01 bis 30 Gew.-%, bevorzugt 0,01 bis 20 Gew.-%, besonders bevorzugt 0,01 bis 10 Gew.-% und insbesondere bevorzugt 0,1 bis 5 Gew.-% enthalten sein, bezogen auf die Zusammensetzung.

Bei den thermoplastischen Polymeren kann es sich zum Beispiel um die folgenden Polymeren, Copolymeren bzw. Mischungen von diesen Polymeren handeln:
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Polyether aus Diglycidylverbindungen, zum Beispiel Diglycidylethern und Diolen, z. B. aus Bisphenol-A-Diglycidylether und Bisphenol-A.
21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PC/Poly(epichlorhydrin).

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Duroplasten und strukturell vernetzten Polymeren kann es sich zum Beispiel um folgende Polymere handeln:
1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
2. Trocknende und nicht-trocknende Alkydharze.
3. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
4. Vernetzbare Acrylharze, die sich von substituierten Acrylsäureestern ableiten, wie z.B. von Epoxyacrylaten, Urethan-acrylaten oder Polyester-acrylaten.
5. Alkyldharze, Polyesterharze und Acrylatharze, die mit Melaminharzen, Harnstoffharzen, Polyisocyanaten oder Epoxidharzen vernetzt sind.
6. Kautschuk auf der Basis von vernetzten Polydienen, zum Beispiel Butadien oder Isopren; Silikonkautschuk.
7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether; Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, Δ₄-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N''-Diglycidyl-N-phenyl-isocyanurat; N,N'-Diglycidylethylenharnstoff; N,N'-Diglycidyl-5,5-dimethyl-hydantoin, N,N'-Diglycidyl-5-isopropyl-hydantoin, N,N-Methylen-bis-(N',N'-diglycidyl-5,5-dimethylhydantoin), 1,3-Bis-(N-glycidyl-5,5-methylhydantoin)-2-hydroxypropan; N,N'-Diglycidyl-5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromarische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantetraglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylgruppen und Epoxiden vorreagierte Addukte.

Als Härter für Epoxidharze kommen saure oder basische Verbindungen Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäreanhydrid, 4-Methyl-3,6-endomethylen-tetrahydrophthalsäureanhydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbernsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigern oder nur mit thermischen Härtungskatalysatoren gehärtet werden. Beispiele sind 3-Ethyl-4-methylimidazol, Triamylammoniumphenolat); Mono- oder Polyphenole (Phenol, Diomenthan, Salicylsäure); Bortrifluorid und seine Komplexe mit organischen Verbindungen wie z. B. Bortrifluorid-Etherkomplexe und BortrifluoridAmin-Komplexe (BF₃-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Der erfindungsgemässen Zusammensetzung können weitere Additive zur Verbesserung der Verarbeitungseigenschaften, der mechanischen, elektrischen und thermischen Eigenschaften, der Oberflächeneigenschaften und der Lichtstabilität einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

Eine bevorzugte Ausführungsform der erfindungsgemässen Zusammensetzung ist dadurch gekennzeichnet, dass sie als Formkörper, Filme, Folien, Fasern, oder als Beschichtungen auf mindestens einer Oberfläche eines Substrats ausgebildet ist.

Ein weiterer Gegenstand der Erfindung ist ein Verfahren zur Herstellung von erfindungsgemässen Zusammensetzungen, das dadurch gekennzeichnet ist, dass man (a) einen CT-Komplex der Formel I einem thermoplastischen Kunststoff einverleibt, (b) einen CT-Komplex der Formel I mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel II oder ein Fulvalenderivat der Formeln III oder IIIa zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung eines Fulvalenderivats der Formeln III oder IIIa beziehungsweise einer Verbindung der Formel II vermischt, das Lösungsmittel entfernt und härtbare Mischungen zu einem duroplastischen oder strukturell vernetzten Kunststoff polymerisiert. Das Herstellungsverfahren kann mit einer Formgebung verbunden sein.

Die Herstellung der erfindungsgemässen Zusammensetzung kann nach in der Kunststofftechnik bekannten Verfahren erfolgen. Bei Formgebungsverfahren für Polymere, zum Beispiel Giessen, Pressverfahren, Spritzgiessen und Extrusion, kann der CT-Komplex selbst unter Bildung von Suspensionen einem Thermoplasten oder mindestens einem Ausgangsstoff für Duroplaste, oder getrennt je einem Ausgangsstoff (zum Beispiel dem Epoxidharz und dem Härter) unter Bildung von Lösungen oder Suspensionen zugegeben werden, wobei nach der Formgebung der CT-Komplex während des Abkühlens in Form von Nadeln auskristallisiert beziehungsweise ausfällt, und die Nadeln ein Netzwerk in einer Polymermatrix bilden.

In einer besonders bevorzugten Ausführungsform ist die erfindungsgemässe Zusammensetzung als Film oder Folie oder als Beschichtung auf mindestens einer Oberfläche eines Substrats ausgebildet. Zur Herstellung solcher Ausführungsformen wird zum Beispiel ein thermoplastisches Polymer oder mindestens ein Ausgangsprodukt für einen Duroplasten oder ein strukturell vernetztes Polymer in einem inerten Lösungsmittel zusammen mit einem CT-Komplex der Formel I suspendiert und /oder gelöst, oder zusammen mit einer Verbindung der Formel II oder einem Fulvalenderivat B gelöst und dann mit einer Lösung des Fulvalenderivats B beziehungsweise einer Verbindung der Formel II versetzt und vermischt, danach mittels bekannter Beschichtungstechniken auf ein gegebenenfalls vorerwärmtes Substrat aufgetragen und dann unter Erwärmen das Lösungsmittel entfernt, wobei vernetzbare Mischungen dann noch ausgehärtet werden können. Freitragende Filme und Folien werden durch Ablösen vom Substrat oder mittels Exausionsverfahren erhalten.

Geeignete Substrate sind zum Beispiel Glas, Metalle, Kunststoffe, mineralische und keramische Werkstoffe, Holz und Papier. Die Substrate können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Geeignete Beschichtungsverfahren sind zum Beispiel Streichen, Walzen, Rakeln, Giessen, Schleudergiessen, Vorhanggiessen und Sprayen. Besonders bevorzugte Verfahren sind Sprayverfahren, weil zum einen sehr dünne und gleichmässige Schichten mit im wesentlichen isotropen, sehr feinmaschigen und homogenen Netzwerken aus Kristallnadeln der CT-Komplexe erhältlich sind, und zum anderen die Grösse der Kristallnadeln und die Maschenweite der Netzwerke durch die Tröpfchengrösse kontrolliert werden kann, selbst wenn man Suspensionen sprüht.

Geeignete inerte Lösungsmittel für Polymere beziehungsweise Ausgangsprodukte für Polymere sind zum Beispiel polare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) und Nitrile (Acetonitril, Propionitril). Geeignet sind auch aromatisch-aliphatische Ether wie zum Beispiel Methyl- oder Ethylphenylether sowie Ketone wie zum Beispiel Aceton, Methylethylketon, Methylpropylketon, Methylbutylketon, Dipropylketon, Dibutylketon und Methylisobutylketon. Geeignete Lösungsmittel für die Verbindungen der Formel II und die Fulvalenderivate B sind zuvor genannt worden.

Die Beschichtungsverfahren können zum Beispiel so durchgeführt werden, dass man die einzelnen Bestandteile getrennt löst und erst vor dem gewählten Beschichtungsverfahren vereinigt. Man kann aber auch die Bestandteile in zwei Lösungen, zum Beispiel Polymerlösung und Fulvalenderivat B oder einer Verbindung der Formel II und Lösung einer Verbindung der Formel II oder eines Fulvalenderivats B gegebenenfalls zusammen mit einem Polymer herstellen, oder alle Bestandteile in einer Lösung vereinigen. Im letzten Fall können die CT-Komplexe schon vor der Beschichtung auskristallisieren, was aber praktisch keinen Einfluss auf die gewünschte Qualität der Beschichtung hat.

Die Lösungen werden zweckmässig erwärmt, zum Beispiel auf 30 bis 200 °C. Es ist vorteilhaft, auch das Substrat zu erwärmen, um die Entfernung des Lösungsmittels zu beschleunigen, die im allgemeinen bei Temperaturen von 50 bis 150 °C, bevorzugt 50 bis 100 °C vorgenommen wird, bis die Beschichtung trocken ist. Sofern die Beschichtungen zu frei tragenden Filmen oder Folien abgelöst werden sollen, kann das Substrat vor der Beschichtung mit Antihaftmitteln behandelt werden.

In einer Herstellvariante für Beschichtungen kann man auch die CT-Komplexe, die als Kristallnadeln ausgebildet sind, in einer Lösung eines Polymeren oder Ausgangsstoffen für Duroplaste suspendieren, dann ein Substrat beschichten und danach das Lösungsmittel entfernen und gegebenenfalls zur Bildung der Duroplaste aushärten. Ferner ist es möglich, pulvrige Trockenmischungen aus Polymerpulvern oder festen Ausgangsstoffen für Duroplaste und den CT-Komplexen herzustellen und diese in gegebenenfalls elektrostatischen Beschichtungsverfahren zu Schichten auf Substraten zu verarbeiten. Auch bei diesen Varianten werden Netzwerke von Kristallnadeln in einer Polymermatrix erhalten.

Es ist auch möglich, reine Schichten von Netzwerken aus Kristallnadeln der CT-Komplexe auf einem Substrat herzustellen, indem man zum Beispiel Lösungen oder Suspensionen der CT-Komplexe in einem Lösungsmittel auf einem Substrat aufbringt und danach das Lösungsmittel verdampft. Solche Schichten können zur Erhöhung der elektrischen Leitfähigkeit elektrochemisch metallisiert werden, zum Beispiel mit Cu, Pt oder Pd. Es kann zweckmässig sein, solche reine Schichten mit einer Schutzschicht aus einem Polymer zu beschichten oder die reinen Schichten nachträglich mit einem Polymer zu umhüllen.

Die Schichtdicken können in einem breiten Rahmen variieren, je nach Wahl des Beschichtungsverfahrens. Mit Sprayverfahren können sehr dünne Schichten erhalten werden, während man mit Streich- und Giessverfahren auch dicke Schichten erzielen kann. Die Schichtdicken können zum Beispiel 0,01 bis 5000 µm, bevorzugt 0,1 bis 1000 µm und besonders bevorzugt 0,1 bis 500 µm betragen.

Die erfindungsgemässe Zusammensetzung ist je nach Wahl des Polymers opak oder transparent und sie weist hervorragende elektrische Eigenschaften auf. So weisen die Beschichtungen und Formkörper überraschend eine ausgezeichnete Entladungsfähigkeit auf, die für heterogene Materialien sonst schwierig oder gar nicht zu erreichen ist. Die Zusammensetzung eignet sich daher besonders zur Verwendung als antistatisch ausgerüstete Formteile für die elektrostatische Abschirmung von Bauteilen oder zur Verwendung als antistatisch ausgerüstete Formkörper. Die hohen elektrischen Leitfähigkeiten ermöglichen ferner die Verwendung als elektrische Leiter, zum Beispiel als Elektroden für Displayelemente oder elektronische Bauteile sowie als Ladungsspeicher in Kondensatoren. Die Zusammensetzungen weisen auch hervorragende mechanische Festigkeiten und mechanische Gebrauchseigenschaften auf. Die Zusammensetzungen können auch bei vergleichsweise niedrigen Temperaturen hergestellt werden und bieten zudem den Vorteil, keine oder nur eine unwesentliche Korrosion bei metallischen Maschinenteilen zu verursachen. Ferner weisen sie gute Stabilitäten gegenüber dem Einfluss von Wärme und/oder Feuchtigkeit auf.

Weitere Gegenstände der Erfindung sind die Verwendung der erfindungsgemässen Charge-Transfer Komplexe der Formel I als elektrische Leiter; die Verwendung der erfindungsgemässen Zusammensetzung als antistatisch ausgerüstete Formteile für die eletrostatische Abschirmung von Bauteilen oder als antistatisch ausgerüstete Formkörper; die Verwendung der erfindungsgemässen Zusammensetzung als elektrische Leiter; die Verwendung der erfindungsgemässen Zusammensetzung als Elektrodenmaterial und die Verwendung der erfindungsgemässen Zusammensetzung in Form von Filmen oder Folien als Ladungsträger in Kondensatoren.

Die nachfolgenden Beispiele erläutern die Erfindung näher.

### A) Herstellungsbeispiele

### Beispiel A1: Herstellung eines CT-Komplexes aus Tetrathiofulvalen und 5,7,12,14-Tetracyanoiminpentacen.

Zu einer 80 °C warmen Lösung von 200 mg (0,460 mMol) 5,7,12,14-Tetracyanoiminpentacen in 200 ml 1,2-Dichlorethan wird eine gleich warme Lösung von 94 mg (0,460 mMol) Tetrathiofulvalen in 10 ml 1,2-Dichlorethan gegeben. Man erhält eine dunkelgelbe Suspension, die zunächst auf Raumtemperatur und dann auf -5°C abgekühlt wird. Der Niederschlag wird abfiltriert, mit CH₂Cl₂ gewaschen, und dann im Hochvakuum getrocknet. Man erhält die Titelverbindung in einer Ausbeute von 197 mg (67%) in Form von dunkelgrünen Kristallnadeln. Die elektrische Leitfähigkeit (Pressling) beträgt 19,0 S/cm. Elementaranalyse gefunden (berechnet): C 59,75 (60,17); H 2,35 (2,21); N 17,48 (17,54); S 19,80 (20,08). Zersetzungstemperatur 256 °C.

### Beispiel A2: Herstellung eines CT-Komplexes aus Tetramethyltetrathiofulvalen und 5,7,12,14-Tetracyanoiminpentacen.

Zu einer 80 °C warmen Lösung von 217 mg (0,500 mMol) 5,7,12,14-Tetracyanoiminpentacen in 200 ml 1,2-Dichlorethan wird eine gleich warme Lösung von 130 mg (0,506 mMol) Tetramethyltetrathiofulvalen in 20 ml 1,2-Dichlorethan gegeben. Man erhält eine dunkelgelbe Suspension, die zunächst auf Raumtemperatur und dann auf -5°C abgekühlt wird. Der Niederschlag wird abfiltriert, mit CH₂Cl₂ gewaschen, und dann im Hochvakuum getrocknet. Man erhält die Titelverbindung in einer Ausbeute von 211 mg (61 %) in Form von dunkelroten Kristallnadeln. Die elektrische Leitfähigkeit (Pressling) beträgt 9,0 S/cm. Elementaranalyse gefunden (berechnet): C 61,83 (61,23); H 3,12 (3,19); N 16,28 (16,13); S 18,05 (18,46). Zersetzungstemperatur 226 °C.

### Beispiel A3: Herstellung eines CT-Komplexes aus Bis-(dithioethylen)tetrathiofulvalen und 5,7,12,14-Tetracyanoiminpentacen.

Zu einer 100 °C warmen Lösung von 100 mg (0,260 mMol) Bis-(dithioethylen)tetrathiofulvalen in 20 ml Anisol wird eine gleich warme Lösung von 57 mg (0,13 mMol) 5,7,12,14-Tetracyanoiminpentacen in 30 ml Anisol gegeben. Man erhält eine rote Suspension, die auf Raumtemperatur abgekühlt wird. Der Niederschlag wird abfiltriert, mit Diethylether gewaschen, und dann im Hochvakuum getrocknet. Man erhält die Titelverbindung in einer Ausbeute von 90 mg (57%) in Form von schwarzen Kristallnadeln. Die elektrische Leitfähigkeit (Pressling) beträgt 1,3x10⁻² S/cm. Elementaranalyse gefunden (berechnet): C 46,45 (45,90); H 2,50 (2,18); N 9,30 (9,31); S 42,43 (42,62).

### Beispiel A4: Herstellung eines CT-Komplexes aus Tetraselenofulvalen und 5,7,12,14-Tetracyanoiminpentacen.

Zu einer 100 °C warmen Lösung von 100 mg (0,260 mMol) Tetraselenofulvalen in 20 ml Anisol wird eine gleich warme Lösung von 111 mg (0,260 mMol) 5,7,12,14-Tetracyanoiminpentacen 30 ml Anisol gegeben. Man erhält eine rote lösung, die auf -5 °C abgekühlt wird. Der Niederschlag wird abfiltriert, mit Diethylether gewaschen, und dann im Hochvakuum getrocknet. Man erhält die Titelverbindung in einer Ausbeute von 90 mg (57%) in Form von dunkelgrünen Kristallnadeln. Die elektrische Leitfähigkeit (Pressling) beträgt 4,8 S/cm. Elementaranalyse gefunden (berechnet): C 46,90 (46,51); H 1,78 (1,71); N 13,56 (13,56); Se 37,80 (38,22).

### B) Anwendungsbeispiele

### Beispiel B1:

Zu einer 70 °C warmen Lösung von 100 mg Polycarbonat und 1,5 mg Tetrathiofulvalen in 8 ml 1,2-Dichlorethan wird eine 70 °C warme Lösung von 4 mg 5,7,12,14-Tetracyanoiminpentacen in 3 ml 1,2-Dichlorethan zugegeben. Aliquote Teile werden auf eine Glasplatte gegossen und dann das Lösungsmittel bei verschiedenen Temperaturen abgedampft. An den erhaltenen Folien werden anschliessend die Leitfähigkeiten gemessen.

| Abdampftemperatur (°C) | Leitfähigkeit (S/cm) |
|---|---|
| 40 | 0.03 |
| 45 | 0,14 |
| 50 | 0,03 |

## Patentansprüche

1. Zusammensetzung, enthaltend a) ein duroplastisches, thermoplastisches oder strukturell vernetztes Polymer und b) einen Charge-Transfer komplex der Formel I in Form eines Netzwerks aus Kristallnadeln in der Polymermatrix,
[A]^{⊖·}B^{⊕} (I),
worin
A eine Verbindung der Formel II oder eine Mischung von Verbindungen der Formel II ist, 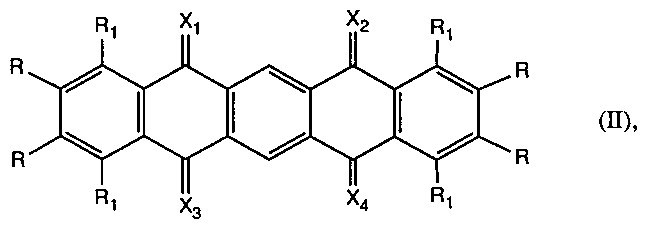 worin die R gleich sind und für H oder C₁-C₄-Alkyl stehen, oder die benachbarten R zusammen -(CH₂)₃- oder -(CH₂)₄- darstellen; R₁ H oder C₁-C₄-Alkyl bedeutet; X₁ =N-CN darstellt und X₂, X₃ und X₄ unabhängig voneinander =O oder =N-CN bedeuten, und
B eine Verbindung der Formel III oder IIIa bedeutet, 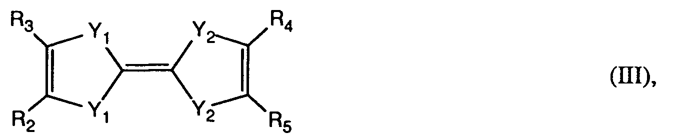 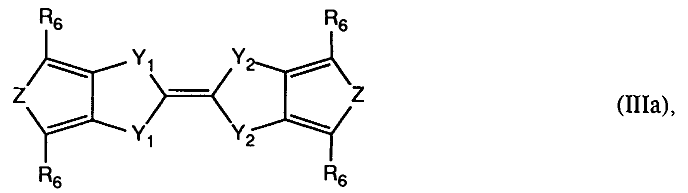 worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder NR₇ bedeutet und R₇ H, C₁-C₆-Alkyl, Phenyl oder Benzyl ist, und R₆ für H, C₁-C₄-Alkyl, Phenyl oder Benzyl steht.

2. Zusammensetzung gemäss Anspruch 1, dadurch gekennzeichnet, dass die Verbindungen der Formeln III und IIIa ein Reduktionspotential von kleiner oder gleich 0,44 V aufweisen, bezogen auf die Standardkalomelelektrode.

3. Zusammensetzung gemäss Anspruch 1, dadurch gekennzeichnet, dass R in Formel II C₁-C₄-Alkyl bedeutet und R₁ für H steht.

4. Zusammensetzung gemäss Anspruch 1, dadurch gekennzeichnet, dass in Formel II R₁ C₁-C₄-Alkyl bedeutet und R für H steht.

5. Zusammensetzung gemäss Anspruch 1, dadurch gekennzeichnet, dass in Formel II R und R₁ in der Bedeutung von Alkyl für Methyl oder Ethyl stehen.

6. Zusammensetzung gemäss Anspruch 1, dadurch gekennzeichnet, dass in Formel II R und R₁ für H, Methyl oder Ethyl stehen.

7. Zusammensetzung gemäss Anspruch 1, dadurch gekennzeichnet, dass in Formel II R und R₁ für H stehen.

8. Zusammensetzung gemäss Anspruch 1, dadurch gekennzeichnet, dass in Formel II X₁ und X₄ =N-CN und X₂ und X₃ =O oder =N-CN, oder X₂ und X₃ =N-CN und X₁ und X₄ -O oder =N-CN bedeuten.

9. Zusammensetzung gemäss Anspruch 1, dadurch gekennzeichnet, dass in Formel II X₁, X₂, X₃ und X₄ =N-CN darstellen.

10. Zusammensetzung gemäss Anspruch 1, dadurch gekennzeichnet, dass in Formel III R₂ und R₃ sowie R₄ und R₅ gleiche Reste darstellen.

11. Zusammensetzung gemäss Anspruch 1, dadurch gekennzeichnet, dass in Formel III R₂ bis R₅ gleiche Reste bedeuten.

12. Zusammensetzung gemäss Anspruch 1, dadurch gekennzeichnet, dass in den Formeln III und IIIa Y₁ und Y₂ je für -S- stehen.

13. Zusammensetzung gemäss Anspruch 1, dadurch gekennzeichnet, dass es sich bei R₂ bis R₅ in Formel III in der Bedeutung von Alkyl-(Z₁)ₙ- um C₁-C₁₂-Alkyl-(Z₁)ₙ- handelt, worin Z₁ und n die in Anspruch 1 angegebenen Bedeutungen haben.

14. Zusammensetzung gemäss Anspruch 1, dadurch gekennzeichnet, dass es sich bei dem Rest Alkyl-(Z₁)ₙ- um Methyl, Ethyl, n- und i-Propyl, n-, i- und t-Butyl, Methylthio, Methylseleno, Ethylthio und Ethylseleno handelt.

15. Zusammensetzung gemäss gemäss Anspruch 1, dadurch gekennzeichnet, dass R₆ in Formel IIIa H oder C₁-C₄-Alkyl bedeutet.

16. Zusammensetzung gemäss gemäss Anspruch 1, dadurch gekennzeichnet, dass Z₁ für -S- und Z₂ für -S- oder -O- stehen.

17. Zusammensetzung gemäss gemäss Anspruch 1, dadurch gekennzeichnet, dass es sich bei den CT-Komplexen der Formel I um solche handelt, worin in Formel II R H, Methyl oder Ethyl bedeutet, R₁ H oder Methyl darstellt und X₁ bis X₄ für =N-CN stehen, und in den Formeln III und IIIa R₂ und R₃ sowie R₄ und R₅ oder R₂ bis R₅ gleich sind und H, lineares oder verzweigtes C₁-C₈-Alkyl-(Z₁)ₙ-, unsubstituiertes oder mit C₁-C₄-Alkyl substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl substituiertes Trimethylen, Tetramethylen, -Z₂-(CH₂)-Z₂-, -Z₂-(CH₂)₂-Z₂-, -Z₁-CH=CH-Z₁ - oder -CH=CH-CH=CH- darstellen, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄Alkyl ist, und R₆ für H oder C₁-C₄-Alkyl steht.

18. Zusammensetzung gemäss gemäss Anspruch 17, dadurch gekennzeichnet, dass es sich bei den CT-Komplexen der Formel I um solche handelt, worin in Formel II R und R₁ H bedeuten, X₁ bis X₄ für =N-CN stehen, und in den Formeln III und IIIa R₂ bis R₅ gleich sind und H, oder lineares oder verzweigtes C₁-C₄-Alkyl-(Z₁)ₙ- darstellen, oder R₂ und R₃ sowie R₄ und R₅ je zusammen Trimethylen, Tetramethylen, -Z₂-(CH₂)-Z₂-, -Z₂-(CH₂)₂-Z₂-, -Z₁-CH=CH-Z₁- oder -CH=CH-CH=CH- bedeuten, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄-Akyl ist, und R₆ für H oder C₁-C₄-Alkyl steht.

19. Zusammensetzung gemäss Anspruch 1, dadurch gekennzeichnet, dass es sich bei den CT-Komplexen der Formel I um solche handelt, worin A 5,7,12,14-Tetracyanoiminpentacen ist und B eine Verbindung der Formel IIIb darstellt, 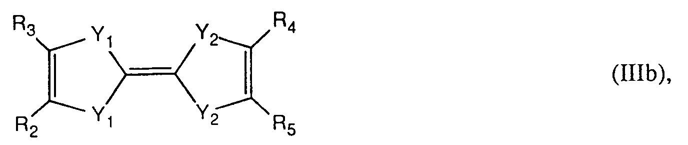 worin Y₁ und Y₂ je für -S- stehen, und R₂, R₃, R₄ und R₅ unabhängig voneinander für H, C₁-C₄-Alkyl oder C₁-C₄-Alkylthio oder R₂ und R₃ sowie R₄ und R₅ zusammen für -CH₂CH₂-S - stehen.

20. Zusammensetzung gemäss Anspruch 19, dadurch gekennzeichnet, dass es sich bei den CT-Komplexen der Formel I um solche handelt, worin A 5,7,12,14-Tetracyanoiminpentacen ist, und B Tetrathiofulvalen, Tetramethyltetrathiofulvalen, Tetraethyltetrathiofulvalen, Dimethyl-diethyl-tetrathiofulvalen, Tetra-n-propyl-tetrathiofulvalen, Tetra-n-butyl-tetrathiofulvalen, Tetra(methylthio)tetrathiofulvalen, Tetra(ethylthio)tetrathiofulvalen, Tetra(n-propylthio)tetrathiofulvalen, Tetra(n-butylthio)tetrathiofulvalen, Dimethyl-dimethylthiotetrathiofulvalen, Diethyl-dimethylthiotetrathiofulvalen, Diethylthio-dimethylthiotetrathiofulvalen und Tetraselenofulvalen bedeutet.

21. Zusammensetzung gemäss Anspruch 20, dadurch gekennzeichnet, dass der Charge-Transfer Komplex in einer Menge von 0,01 bis 30 Gew.-% enthalten ist, bezogen auf die Zusammensetzung.

22. Zusammensetzung gemäss Anspruch 21, dadurch gekennzeichnet, dass der Charge-Transfer Komplex in einer Menge von 0,01 bis 10 Gew.-% enthalten ist.

23. Zusammensetzung gemäss Anspruch 1, dadurch gekennzeichnet, dass es sich bei dem thermoplastischen Polymer um Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol handelt.

24. Zusammensetzung gemäss Anspruch 1, dadurch gekennzeichnet, dass es sich bei dem duroplastischen Polymer um ein Epoxidharz handelt.

25. Zusammensetzung gemäss Anspruch 1, dadurch gekennzeichnet, dass sie als Formkörper, Filme, Folien, Fasern, oder als Beschichtungen auf mindestens einer Oberfläche eines Subsaats ausgebildet ist.

26. Zusammensetzung gemäss Anspruch 25, dadurch gekennzeichnet, dass die Schichtdikke der Beschichtungen 0.01 bis 5000 µm beträgt.

27. Zusammensetzung gemäss Anspruch 26, dadurch gekennzeichnet, dass die Schichtdikke der Beschichtungen 0,1 bis 1000 µm beträgt.

28. Verfahren zur Herstellung von Zusammensetzungen gemäss Anspruch 1, dadurch gekennzeichnet, dass man (a) einen CT-Komplex der Formel I einem thermoplastischen Kunststoff einverleibt, (b) einen CT-Komplex der Formel I mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel II oder ein Fulvalenderivat B zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung eines Fulvalenderivats B beziehungsweise einer Verbindung der Formel II vermischt, das Lösungsmittel entfernt und härtbare Mischungen zu einem duroplastischen oder strukturell vernetzten Kunststoff polymerisiert.

29. Verfahren gemäss Anspruch 28, dadurch gekennzeichnet, dass es mit einer Formgebung verbunden ist.

30. CT-Komplexe der Formel Ia
[A]^{⊖·}B^{⊕} (Ia),
worin
A eine Verbindung der Formel II oder eine Mischung von Verbindungen der Formel II ist, 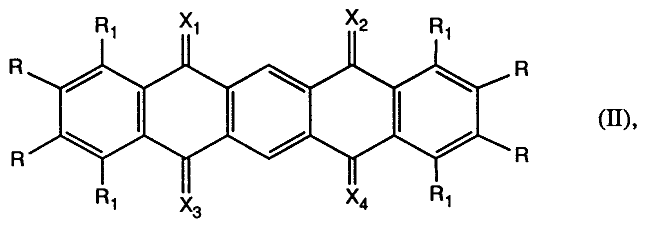 worin die R gleich sind und für H oder C₁-C₄-Alkyl stehen, oder die benachbarten R zusammen -(CH₂)₃- oder -(CH₂)₄- darstellen; R₁ H oder C₁-C₄-Alkyl bedeutet; X₁ =N-CN darstellt und X₂, X₃ und X₄ unabhängig voneinander =O oder =N-CN bedeuten, und
B eine Verbindung der Formel III oder IIIa bedeutet, 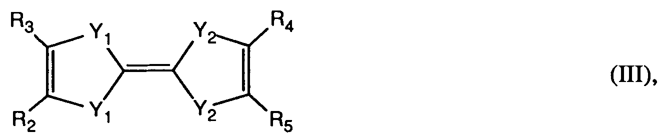 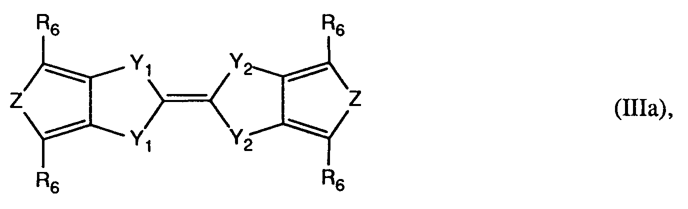 worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder NR₇ bedeutet und R₇ H, C₁-C₆-Alkyl, Phenyl oder Benzyl ist, und R₆ für H, C₁-C₄-Alkyl, Phenyl oder Benzyl steht, mit Ausnahme von [(5,7,12,14-Tetracyanoimintetracen)(Tetrathiofulvalen)].

31. CT-Komplexe der Formel Ia mit Ausnahme von [(5,7,12,14-Tetracyanoimintetracen)(Tetrathiofulvalen)] gemäss Anspruch 30, dadurch gekennzeichnet, dass sie in Form von nadelförmigen Kristallen vorliegen.

32. Verwendung der Charge-Transfer Komplexe der Formel Ia gemäss Anspruch 30 als elektrische Leiter.

33. Verwendung der Zusammensetzung gemäss Anspruch 1 als antistatisch ausgerüstete Formteile für die elektrostatische Abschirmung von Bauteilen oder als antistatisch ausgerüstete Formkörper.

34. Verwendung der Zusammensetzung gemäss Anspruch 1 als elektrische Leiter.

35. Verwendung der Zusammensetzung gemäss Anspruch 1 als Elektrodenmaterial.

36. Verwendung der Zusammensetzung gemäss Anspruch 1 in Form von Filmen oder Folien als Ladungsträger in Kondensatoren.
